# EUROPÄISCHE PATENTSCHRIFT

(11) **EP 2 772 762 B1**
(45) Veröffentlichungstag und Bekanntmachung des Hinweises auf die Patenterteilung: **30.11.2016**
(21) Anmeldenummer: 13157111.9
(22) Anmeldetag: 28.02.2013
(51) Int. Cl.: G01N 33/86

(54) **Verfahren zur Bestimmung der Protein S-Aktivität**
Method for determining protein S activity
Procédé de détermination de l'activité de protéines S

(43) Veröffentlichungstag der Anmeldung: 03.09.2014
(73) Patentinhaber: Siemens Healthcare Diagnostics Products GmbH, 35041 Marburg (DE)
(72) Erfinder: Patzke, Juergen Dr., 35043 Marburg (DE); Meuer, Joern Dr., 35094 Lahntal (DE); Wissel, Thomas Dr., 35094 Lahntal (DE)

(56) Entgegenhaltungen:
- EP-A2- 0 942 284
- US-A1- 2003 073 070
- L. F. A. MAURISSEN ET AL: "Re-evaluation of the role of the protein S-C4b binding protein complex in activated protein C-catalyzed factor Va-inactivation", BLOOD, Bd. 111, Nr. 6, 15. März 2008 (2008-03-15) , Seiten 3034-3041, XP055067156, ISSN: 0006-4971, DOI: 10.1182/blood-2007-06-089987
- MALM J ET AL: "INHIBITION OF HUMAN VITAMIN-K-DEPENDENT PROTEIN-S-COFACTOR ACTIVITY BY A MONOCLONAL ANTIBODY SPECIFIC FOR A CA2+-DEPENDENT EPITOPE", EUROPEAN JOURNAL OF BIOCHEMISTRY, BLACKWELL PUBLISHING, BERLIN, DE, Bd. 165, Nr. 1, 1. Januar 1987 (1987-01-01), Seiten 39-45, XP008032522, ISSN: 0014-2956, DOI: 10.1111/J.1432-1033.1987.TB11191.X
- SHEN L ET AL: "FACTOR V AND PROTEIN S AS SYNERGISTIC COFACTORS TO ACTIVATED PROTEIN C IN DEGRADATION OF FACTOR VIIIA", JOURNAL OF BIOLOGICAL CHEMISTRY, AMERICAN SOCIETY FOR BIOCHEMISTRY AND MOLECULAR BIOLOGY, US, Bd. 269, Nr. 29, 22. Juli 1994 (1994-07-22), Seiten 18735-18738, XP002004743, ISSN: 0021-9258
- HACKENG TILMAN M ET AL: "Protein S binding to human endothelial cells is required for expression of cofactor activity for activated protein C", JOURNAL OF BIOLOGICAL CHEMISTRY, Bd. 268, Nr. 6, 1993, Seiten 3993-4000, XP055067200, ISSN: 0021-9258
- GUERMAZI S ET AL: "Les deficits congenitaux en proteineS ; difficultes diagnostiques", PATHOLOGIE ET BIOLOGIE, L'EXPANSION SCIENTIFIQUE FRANCAISE, PARIS, FR, Bd. 57, Nr. 6, 1. September 2009 (2009-09-01), Seiten 483-487, XP026574739, ISSN: 0369-8114, DOI: 10.1016/J.PATBIO.2008.04.016 [gefunden am 2008-06-25]

## Beschreibung

Die Erfindung liegt auf dem Gebiet der Gerinnungsdiagnostik und betrifft ein in vitro Verfahren zur Bestimmung der Protein S-Aktivität.

Das Blutgerinnungssystem erfüllt zwei gegensätzliche Aufgaben: einerseits sorgt es durch antikoagulatorische Mechanismen dafür, dass ein kontinuierlicher Blutstrom aufrecht erhalten wird; andererseits gewährleistet es im Falle einer Gefäßverletzung durch prokoagulatorische Mechanismen, dass ein Wundverschluss stattfindet.

Bei der Aktivierung des Gerinnungssystems infolge einer Gefäßverletzung entsteht über ein kaskadenartiges System aktivierender Proteasen letztlich die aktive Protease Thrombin (Faktor IIa). Die anfänglich nur sehr langsame Thrombinbildung wird durch Thrombin selbst beschleunigt, indem es die Kofaktoren Faktor V und VIII durch proteolytische Spaltung aktiviert. Die aktivierten Kofaktoren Faktor Va und VIIIa bilden mit den Proteasen Faktor Xa und IXa auf Phospholipidoberflächen einen Enzym-Kofaktor-Komplex, dessen Thrombin-aktivierende Aktivität ca. 1000 fach höher ist als die Aktivität der singulären Proteasen. Durch diese positive Rückkopplung kommt es zu einer explosionsartigen Entstehung großer Thrombinmengen. Thrombin setzt Fibrinogen zu Fibrin um, welches polymerisiert und zum Wundverschluss führt.

Um eine lebensbedrohliche Ausweitung der Gerinnung zu verhindern, die zu einem Verschluss des Gefäßsystems im Körper führen würde, muss sowohl das gebildete Thrombin inaktiviert werden als auch die Bildung weiteren Thrombins unterbunden werden. Aktives Thrombin wird durch Komplexbildung mit Thrombin-Inhibitoren wie z.B. Antithrombin neutralisiert. Die Bildung weiteren Thrombins wird durch das Thrombin selbst gedrosselt. Thrombin bindet an das Membranprotein Thrombomodulin. Der zellständige Thrombin-Thrombomodulin-Komplex aktiviert das Inhibitor-Protein Protein C (PC) zu Protein Ca (aktiviertes Protein C, APC). APC inaktiviert die Faktoren Va und VIIIa und drosselt damit die Thrombinbildung. APC benötigt für seine optimale gerinnungsinhibierende Wirkung unter anderem Protein S als Kofaktor.

Protein S ist ein Vitamin K-abhängiges Plasmaprotein. Im Blut ist Protein S zum Teil an C4b-binding-Protein (C4b-BP) gebunden. Nur ca. 40 % des vorhandenen Protein S liegen als ungebundenes, freies Protein S vor. Nur das freie Protein S stimuliert die Inaktivierung von Faktor Va und VIIIa durch APC. Diese gerinnungsinhibierende Aktivität des Protein S wird auch als Protein Ca-Kofaktor-Aktivität oder APC-Kofaktor-Aktivität bezeichnet.

Neben der Protein Ca-Kofaktor-Aktivität, die zur Inaktivierung von Faktor Va und VIIIa führt, kann freies Protein S auch direkt den prokoagulatorischen Prothrombinasekomplex und den prokoagulatorischen Tenasekomplex hemmen, indem es an Faktor Va und an Faktor Xa bindet. Diese zweite gerinnungsinhibierende Aktivität des Protein S wird auch als APC-unabhängige Aktivität bezeichnet (van Wijnen, M. et al., A plasma coagulation assay for an activated protein C-independent anticoagulant activity of protein S. Thromb Haemost 1998, 80: 930-035).

Protein S hat demnach eine wichtige Inhibitorfunktion im Gerinnungssystem. Ein Protein S-Mangel oder eine verminderte Protein S-Aktivität sind anerkannte Risikofaktoren für eine Thrombophilie. Insbesondere die Aktivität von Protein S ist für die Abschätzung eines Thromboserisikos ein wichtiger Marker.

Die Bestimmung der Protein S-Aktivität im klinischen Labor erfolgt häufig über die Bestimmung der Protein Ca-Kofaktor-Aktivität des Protein S. Dazu wird die Probe üblicherweise mit einer standardisierten Menge von aktiviertem Protein C vermischt, und es wird die Gerinnung aktiviert, wodurch Faktor Va entsteht, der durch das zugegebene aktivierte Protein C inhibiert wird. Das in der Probe enthaltene Protein S wirkt als Kofaktor für das aktivierte Protein C. Je höher die Protein S-Aktivität in der Probe ist, desto länger ist die Gerinnungszeit des Reaktionsansatzes. Bekannte, kommerziell erhältliche Teste sind z.B. der Protein S Ac Test von Siemens Healthcare Diagnostics, Marburg, Deutschland oder der STA Protein S Clotting Test von Diagnostica Stago, Frankreich.

Problematisch ist, dass verschiedene Einflussgrößen die Bestimmung der Protein Ca-Kofaktor-Aktivität des Protein S beeinträchtigen können und zu falschen Messergebnissen führen können.

Eine bekannte Einflussgröße ist eine häufig vorkommende Mutation des Faktor V an der Protein Ca-Spaltstelle des Faktor V (Faktor V Leiden), die dazu führt, dass der Faktor Va resistent gegen aktiviertes Protein C ist (APC-Resistenz), d.h. dass er nicht mehr von Protein Ca inaktiviert werden kann. In Proben von Patienten mit einer (unerkannten) Faktor V Leiden-Mutation kann Protein S seine Protein Ca-verstärkende Wirkung nicht entfalten, die Gerinnungsreaktion schreitet ungehindert voran, so dass eine falsch zu niedrige Protein S-Aktivität bestimmt wird. Zum Ausschluss der Störgröße Faktor V Leiden-Mutation wird im Stand der Technik vorgeschlagen, dass der Probe bereits aktivierter Faktor Va zugegeben wird (Wolf, M. et al., A new functional assay for human protein S activity using activated factor V as substrate. Thromb Haemost. 1989, 62(4): 1144-1145 und STA Protein S Clotting Test).

Eine andere bekannte Einflussgröße ist das Vorhandensein von Thrombozyten in der Probe. In Plasmaproben, die durch unsachgemäße Bearbeitung mehr als 10.000 Thrombozyten/µL enthalten, besteht die Gefahr, dass eine falsch zu niedrige Protein S-Aktivität bestimmt wird (Patzke, J. et al., Characterization of a protein S assay measuring the APC cofactor activity. J Lab Med 2007, 31(6): 262-272).

Noch eine andere bekannte Einflussgröße sind in der Patientenprobe enthaltene Antiphospholipid-Antikörper, insbesondere solche, die die in vitro Gerinnungszeit der Patientenprobe beeinträchtigen (Lupus Antikoagulanzien). In Proben von Patienten mit einem (unerkannten) Antiphosholipid-Syndrom kann die Gerinnungszeit-verlängernde Wirkung der Lupus Antikoagulanzien die Gerinnungszeit-verlängernde Wirkung des Protein S überlagern, so dass eine falsch zu hohe Protein S-Aktivität bestimmt wird.

Die Aufgabe der vorliegenden Erfindung besteht darin, bekannte Verfahren zur Bestimmung der Protein Ca-Kofaktor-Aktivität des Protein S, bei denen die Probe mit aktiviertem Protein C oder einer Substanz zur Aktivierung von Protein C und mit Faktor Va oder einer Substanz zur direkten oder indirekten Aktivierung von Faktor V zu einem Reaktionsansatz vermischt wird und die Gerinnungsreaktion im Reaktionsansatz gemessen wird, so zu verbessern, dass der Einfluss jedweder Störgröße elimiert wird, so dass die wahre Protein S-Aktivität der Probe spezifisch bestimmt werden kann.

Die Aufgabe wird dadurch gelöst, dass die Probe in einem zweiten Reaktionsansatz zusätzlich zu denselben Komponenten, die zur Bereitsstellung des ersten Reaktionsansatzes verwendet werden, mit einem Inhibitor, der die Protein Ca-Kofaktor-Aktivität von Protein S inhibiert, vermischt wird und die Gerinnungsreaktion im Reaktionsansatz gemessen wird und dann die Protein Ca-Kofaktor-Aktivität von Protein S bestimmt wird, indem der Quotient aus der Gerinnungsreaktion, die im ersten Reaktionsansatz gemessen wurde, und der Gerinnungsreaktion, die im zweiten Reaktionsansatz gemessen wurde, gebildet wird.

Die Zugabe eines Inhibitors, der die Protein Ca-Kofaktor-Aktivität von Protein S inhibiert, zum zweiten Reaktionsansatz bewirkt, dass die Protein S-Aktivität im Reaktionsansatz gleich oder nahe 0 % ist, während der Einfluss etwaiger Störgrößen in der Probe, wie z.B. Faktor V Leiden, zu hohe Thrombozytenzahl oder Antiphospholipid-Antikörper, auf die Gerinnungsreaktion des Reaktionsansatzes unverändert bleibt. Im zweiten Reaktionsansatz wird also nur der Einfluss etwaiger Störgrößen gemessen. Durch Quotientenbildung der Testergebnisse des ersten und zweiten Reaktionsansatzes wird der Einfluss etwaiger Störgrößen eliminiert, und die wahre Protein S-Aktivität ist quantifizierbar.

Unter einem "Inhibitor, der die Protein Ca-Kofaktor-Aktivität von Protein S inhibiert" ist jede Substanz zu verstehen, die zumindest die Protein Ca-Kofaktor-Aktivität von Protein S inhibiert, wie z.B. monoklonale Protein S-bindende Antikörper oder Protein S-bindende Fragmente monoklonaler Antikörper, die durch geeignete, bekannte Verfahren zur Herstellung von monoklonalen Antikörpern gewonnen wurden und selektiert wurden nach ihrer Fähigkeit die Protein Ca-Kofaktor-Aktivität von Protein S zu inhibieren (siehe auch Beispiel 1). Eine andere geeignete Substanz ist C4b-binding-Protein (C4b-BP), das freies Protein S bindet und dadurch inaktiviert.

Ein bevorzugter Inhibitor, der die Protein Ca-Kofaktor-Aktivität von Protein S inhibiert, ist ein monoklonaler Antikörper, der an Protein S bindet und der von der Hybridomazelllinie 96-168/01 produziert wird. Diese Hybridomazelllinie wurde am 07.11.2012 beim Leibniz-Institut DSMZ- Deutsche Sammlung von Mikroorganismen und Zellkulturen GmbH, Inhoffenstraße 7B, 38124 Braunschweig, Deutschland, unter der Eingangsnummer DSM ACC3188 hinterlegt. Weiterhin bevorzugt sind Protein S-bindende Fragmente des monoklonalen Antikörpers, der von der Hybridomazelllinie 96-168/01 produziert wird.

Ein weiterer in der Anmeldung beschriebener Gegenstand ist die Hybridomazelllinie, die bei der DSMZ unter der Eingangsnummer DSM ACC3188 hinterlegt wurde sowie der Antikörper, der von der Hybridomazelllinie DSM ACC 3188 produziert wird, sowie Protein S-bindende Fragmente davon. Unter einer "Probe" ist im Sinne der Erfindung ein Material zu verstehen, welches das zu bestimmende Protein S vermutlich enthält. Der Begriff Probe bezieht sich auf menschliche oder tierische Körperflüssigkeiten, vornehmlich Blut und Plasma, bevorzugt plättchenarmes Plasma.

In Ausführungsformen des erfindungsgemäßen Verfahrens, bei denen die Probe mit aktiviertem Protein C vermischt wird, wird vorzugsweise gereinigtes humanes Protein Ca verwendet. Alternativ kann die Probe mit einer Substanz zur Aktivierung von Protein C vermischt werden, die das in der Probe und/oder etwaiges der Probe hinzugefügtes Protein C aktivieren kann. Geeignete Substanzen zur Aktivierung von Protein C sind beispielsweise Thrombin, bevorzugt humanes oder bovines Thrombin, oder ein Protein C-Aktivator aus Schlangengift, bevorzugt aus Schlangengift der Gattung Agkistrodon contortrix.

In Ausführungsformen des erfindungsgemäßen Verfahrens, bei denen die Probe mit Faktor Va versetzt wird, wird vorzugsweise gereinigter humaner oder boviner Faktor Va oder gereinigter Faktor Va aus Kaninchen verwendet. Alternativ dazu kann die Probe mit einer Substanz zur direkten oder indirekten Aktivierung von Faktor V vermischt werden. Zur direkten Aktivierung von Faktor V eignen sich insbesondere Thrombin, bevorzugt humanes oder bovines Thrombin, oder ein Faktor V-Aktivator aus Schlangengift, bevorzugt der Faktor V-Aktivator aus dem Schlangengift der Gattung Vipera russelli. Zur indirekten Aktivierung von Faktor V eignen sich insbesondere Substanzen aus der Gruppe der Kontaktphaseaktivatoren, wie beispielsweise Kaolin, Silica, Glas oder Ellagsäure, oder Thromboplastin. Eine weitere Substanz zur indirekten Aktivierung von Faktor V ist ein Prothrombin-Aktivator , bevorzugterweise der Prothrombin-Aktivator aus dem Schlangengift der Gattung Echis carinatus. Noch eine weitere Substanz zur indirekten Aktivierung von Faktor V ist ein Faktor X-Aktivator aus Schlangengift, bevorzugt der Faktor X-Aktivator aus dem Schlangengift der Gattung Vipera russelli.

In einer weiteren Ausführungsform des erfindungsgemäßen Verfahrens kann die Probe zusätzlich mit einem Protein S-Mangelplasma vermischt werden. Dies hat den Vorteil, dass etwaige andere Faktorenmängel in der zu untersuchenden Patientenprobe ausgeglichen werden und keinen Einfluss auf das Testergebnis haben.

Die Gerinnungsreaktion im ersten und im zweiten Reaktionsansatz kann durch Bestimmung des Gerinnungszeitpunkts gemessen werden. Dazu wird die Zeit vom Zeitpunkt der Zugabe von Faktor Va oder der Substanz zur direkten oder indirekten Aktivierung von Faktor V
zur Probe bzw. zum Reaktionsansatz bis zur Bildung eines fassbaren Fibringerinnsels in Sekunden gemessen. Alternativ kann ein chromogenes Substrat für Thrombin verwendet werden und die Zeitspanne ab der Zugabe von Faktor Va oder der Substanz zur direkten oder indirekten Aktivierung von Faktor V zur Probe bis zur Erreichung einer definierten Absorptionsänderungsgeschwindigkeit gemessen werden.

Die Gerinnungszeit kann durch manuelle oder automatische Methoden bestimmt werden. Bei der automatischen Bestimmung ist die Messung einer mechanischen oder einer optischen Eigenschaft des Reaktionsansatzes, z.B. der Viskosität oder Trübung, geeignet. In Fällen automatischer Messung wird üblicherweise eine Eigenschaft des Reaktionsansatzes kontinuierlich gemessen und aus der zeitabhängigen Änderung der Eigenschaft kann mit Hilfe von Auswerteverfahren die Gerinnungszeit als Endpunkt bestimmt werden.

Die Protein Ca-Kofaktor-Aktivität von Protein S wird erfindungsgemäß bestimmt, indem der Quotient aus der Gerinnungsreaktion, die im ersten Reaktionsansatz in Abwesenheit einer spezifischen Inhibitors der Protein Ca-Kofaktor-Aktivität von Protein S gemessen wurde, und der Gerinnungsreaktion, die im zweiten Reaktionsansatz in Anwesenheit eines spezifischen Inhibitors der Protein Ca-Kofaktor-Aktivität von Protein S gemessen wurde, gebildet wird. Je größer der ermittelte Quotient ist, desto höher ist die Protein Ca-Kofaktor-Aktivität von Protein S in der Probe. Durch den Vergleich des für eine Patientenprobe ermittelten Quotienten mit einer geeigneten Kalibrationskurve lässt sich die Protein S-Aktivität bestimmen.

### Figurenbeschreibung

**Figur 1**

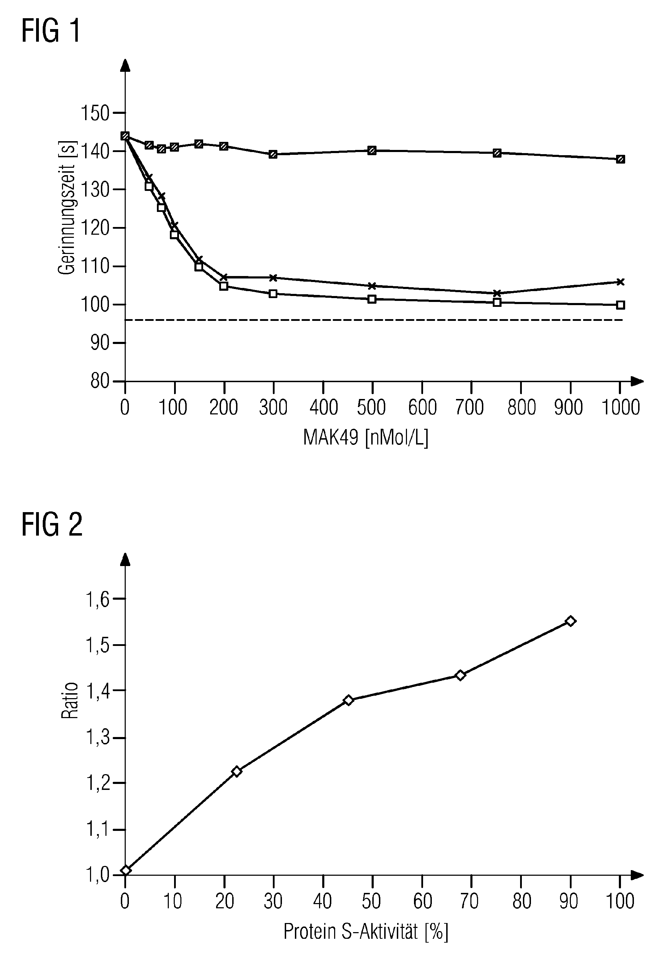

   Figur 1 zeigt die Hemmung der Protein Ca-Kofaktor-Aktivität von Protein S in Normalplasma durch den monoklonalen Antikörper MAK49 (ungefüllte Rechtecke) im Vergleich zur Protein Ca-Kofaktor-Aktivität von Protein S in Normalplasma ohne Inhibitor (gefüllte Rechtecke). Gerinnungszeiten um 143 s stehen für 100 % Protein S-Aktivität, Gerinnungszeiten um 96 s für 0 % Protein S-Aktivität (Protein S Mangelplasma, gestrichelte Linie). Ab einer Konzentration von etwa 300 bis 500 nM MAK49 im Reaktionsansatz wird eine maximale Hemmung der Protein Ca-Kofaktor-Aktivität von Protein S erreicht. Unter "bereinigter Hemmung" (Sternchen) versteht sich die Hemmung, die ausschließlich auf die Anwesenheit von MAK49 zurückzuführen ist. Die verdünnungsbedingte scheinbare Hemmung wurde durch Zugabe entsprechender Mengen Puffer ohne MAK49 (gefüllte Rechtecke) ermittelt, und die Gesamthemmung (ungefüllte Rechtecke) wurde um die entsprechenden Werte bereinigt.
**Figur 2**
   Figur 2 zeigt eine Kalibrationskurve für den Quotienten (Ratio) aus den Gerinnungszeiten in Abwesenheit und in Anwesenheit des Protein S-inhibierenden monoklonalen Antikörpers MAK49 gegen die Protein S-Aktivität(in % der Norm). Die Kalibrationskurve dient dem direkten Ableiten von Protein S-Aktivitäten in % der Norm aus dem Ergebnis des erfindungsgemäßen Verfahrens mit Quotientenbildung einer unbekannten Probe.
**Figur 3**

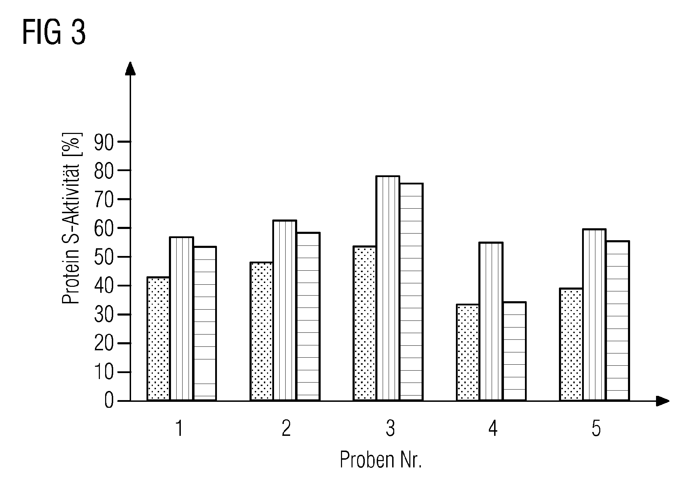

   Figur 3 zeigt für Proben von 5 Spendern mit homozygoter FV Leiden Mutation die Protein Ca-Kofaktor-Aktivität von Protein S (Gerinnungszeit) in Abwesenheit des Inhibitors MAK49 (gepunkteter Balken), die Menge an freiem Protein S-Antigen (längsgestreifte Balken) und den Quotienten aus der Protein Ca-Kofaktor-Aktivität von Protein S (Gerinnungszeit) gemessen in Abwesenheit des MAK49 und der Protein Ca-Kofaktor-Aktivität von Protein S (Gerinnungszeit) gemessen in Anwesenheit des MAK49 (quergestreifte Balken). Die Probe Nr. 4 stammt von einem Patienten, der zusätzlich mit einem oralen Antikoagulanz behandelt wurde.

### Beispiele

### Beispiel 1: Herstellung eines monoklonalen anti-Protein S-Antikörpers, der die Protein Ca-Kofaktor-Aktivität von Protein S inhibiert

Gereinigtes humanes Protein S wurde als Immunisierungsantigen verwendet. Sechs Mäusen wurden jeweils 20 µg Immunisierungantigen (Protein S) intraperitoneal injiziert. Nach mehreren Wochen wurden die Mäuse getötet, und es wurden die Milzen entnommen und Einzelzellsuspensionen hergestellt. Die Milzzellen wurden mit Myelomzellen (Sp2/0) fusioniert, und die so erhaltenen Hybridomazellen wurden in Mikrotiter-Zellkulturplatten überführt. Die Spezifität der in den Zellkulturüberstand abgegebenen Antikörper wurde in einem ersten Schritt mit Hilfe von mit humanem Protein S beschichteten Mikrotiterplatten (Beschichtung 1 µg/ml = 0,15 pg/Vertiefung) getestet. In jede Vertiefung wurde ein Aliquot eines Zellkulturüberstandes pipettiert. Nach einer Inkubationszeit wurde der Überstand entfernt, und jede Vertiefung wurde mit Puffer gewaschen. Anschließend wurde in jede Vertiefung eine Lösung enthaltend einen Peroxidasegekoppelten anti-Maus-IgG-Antikörper pipettiert. Nach einer Inkubationszeit und Waschen der Vertiefungen mit Waschpuffer wurde ein Aliquot einer chromogenen TMB-Lösung in jede Vertiefung gegeben, und die Farbreaktion wurde bei 405 nm gemessen.

Die nach der Klonierung erhaltenen monoklonalen Antikörper, die spezifisch an Protein S binden, wurden anschließend auf ihre Fähigkeit, die Protein Ca-Kofaktor-Aktivität von Protein S zu inhibieren, getestet. Dazu wurden steigende Konzentrationen der monoklonalen anti-Protein S-Antikörper zu Normalplasma gegeben, und die Protein Ca-Kofaktor-Aktivität von Protein S wurde in den Plasmaproben gemessen (zur Messung der Protein Ca-Kofaktor-Aktivität von Protein S siehe Beispiel 2). Parallel wurde gereinigtes C4b-binding-Protein (C4b-BP) zu Normalplasma gegeben, und die Protein Ca-Kofaktor-Aktivität von Protein S wurde gemessen.

Antikörper, mit denen eine Inhibition der Protein S-Aktivität erzielbar ist, die der Inhibition der Protein S-Aktivität entspricht, die durch 100 nMol/L C4b-binding-Protein (C4b-BP) in Normalplasma bewirkt wird, eignen sich als Inhibitoren der Protein Ca-Kofaktor-Aktivität von Protein S. Ein auf diese Weise identifizierter monoklonaler Antikörper ("MAK49") wird von der Hybridomazelllinie 96-168/01 produziert. Diese Hybridomazelllinie wurde am 07.11.2012 beim Leibniz-Institut DSMZ- Deutsche Sammlung von Mikroorganismen und Zellkulturen GmbH, Inhoffenstraße 7B, 38124 Braunschweig, Deutschland, unter der Eingangsnummer DSM ACC3188 hinterlegt.

Figur 1 zeigt die Hemmung der Protein Ca-Kofaktor-Aktivität von Protein S in Normalplasma durch den monoklonalen Antikörper MAK49 (ungefüllte Rechtecke) im Vergleich zur Protein Ca-Kofaktor-Aktivität von Protein S in Normalplasma ohne Inhibitor (gefüllte Rechtecke). Die Gerinnungszeit wurde wie in Beispiel 2 beschrieben gemessen. Gerinnungszeiten um 143 s stehen für 100 % Protein S-Aktivität, Gerinnungszeiten um 96 s für 0 % Protein S-Aktivität (Protein S Mangelplasma, gestrichelte Linie). Ab einer Konzentration von etwa 300 bis 500 nM MAK49 im Reaktionsansatz wird eine maximale Hemmung der Protein Ca-Kofaktor-Aktivität von Protein S erreicht. Unter "bereinigter Hemmung" (Sternchen) versteht sich die Hemmung, die ausschließlich auf die Anwesenheit von MAK49 zurückzuführen ist. Die verdünnungsbedingte scheinbare Hemmung wurde durch Zugabe entsprechender Mengen Puffer ohne MAK49 (gefüllte Rechtecke) ermittelt, und die Gesamthemmung (ungefüllte Rechtecke) wurde um die entsprechenden Werte bereinigt.

### Beispiel 2: Kalibration der Protein S-Aktivität von verschiedenen Proben mit bekannter Protein S-Aktivität gegen den Quotienten aus den Gerinnungszeiten der Proben in Abwesenheit und in Anwesenheit eines Protein S-Inhibitors

### Verfahren zur Bestimmung der Protein Ca-Kofaktor-Aktivität von Protein S

Zur Bestimmung der Protein Ca-Kofaktor-Aktivität von Protein S in Abwesenheit bzw. in Anwesenheit eines Inhibitors, der die Protein Ca-Kofaktor-Aktivität von Protein S inhibiert, wurden 95 µl Probe mit 5 µl Puffer bzw. mit 5 µl Inhibitor-Lösung (10 µM MAK49) vermischt und für ca. drei Minuten inkubiert. Die Proben wurden dann 1:7 mit Protein S-Mangelplasma verdünnt. 16 µL dieser Verdünnung wurden nochmals mit 27 µL Protein S-Mangelplasma vermischt, und nach 20 Sekunden Inkubation wurden 58 µL eines APC-Reagenzes enthaltend humanes aktiviertes Protein C (APC) und Calciumchlorid zugegeben. Nach weiteren 110 Sekunden Inkubation wurden zum Starten der Gerinnungsreaktion 145 µL eines Aktivatorreagenzes zugegeben, welches Russells Viper Venom (RVV, Gift von Vipera russelli) und Phospholipide zur Aktivierug von Faktor X zu Faktor Xa enthält, welcher wiederum Faktor V zu Faktor Va aktiviert.

Die Bestimmung der Protein S-Aktivität erfolgt nach folgendem Prinzip: APC spaltet proteolytisch Faktor Va, der bei der Aktivierung der Gerinnungskaskade durch RVV entsteht. Protein S wirkt dabei als Kofaktor, der die Reaktion beträchtlich beschleunigt. Dadurch kommt es zu einer mit der Aktivität an Protein S in der Probe proportional zunehmenden Gerinnungszeit. Der Zusatz von Mangelplasma sorgt für eine ausreichende Versorgung des Ansatzes mit Fibrinogen, Faktor V und den anderen benötigten Gerinnungsfaktoren. Die Gerinnung wird auf der Stufe des Faktors X durch den Faktor X-Aktivator aus RVV angestoßen. Faktor Xa bildet unter Vermittlung des noch verbliebenen Faktors Va Thrombin aus Prothrombin. Thrombin setzt schließlich Fibrinogen um.

Der Gerinnungszeitpunkt wurde optisch bestimmt.

Es wurden Plasmaproben mit unterschiedlichen Protein S-Konzentrationen hergestellt, indem humanes Normalplasma mit einer Protein S-Aktivität von 90 % der Norm mit Protein S-Mangelplasma mit einer Protein S-Aktivität von 0 % vermischt wurde (Plasmaproben mit 90 %, 67,5 %, 45 %, 22,5 % und 0 % Protein S-Aktivität), und die Proben wurden mit dem vorschriebenen Verfahren untersucht.

Für jede Probe wurde die Gerinnungszeit in Abwesenheit und in Anwesenheit des Protein S-inhibierenden monoklonalen Antikörpers MAK49, der die Protein Ca-Kofaktor-Aktivität von Protein S inhibiert, ermittelt, und es wurde der Quotient aus der Gerinnungszeit gemessen in Abwesenheit des MAK49 und der Gerinnungszeit gemessen in Anwesenheit des MAK49 gebildet. Die so ermittelten Quotienten wurden gegen die bekannte Protein S-Aktivität der Proben aufgetragen, und die resultierende Kurve diente als Kalibrationskurve für die Messung von Proben mit unbekannten Protein S-Aktivitäten. Die Kalibrationskurve ist in Figur 2 gezeigt.

### Beispiel 3: Bestimmung der Protein Ca-Kofaktor-Aktivität von Protein S in Proben von Spendern mit homozygoter FV Leiden-Mutation

Gefrorene Plasmaproben von fünf Spendern mit homozygoter FV Leiden-Mutation wurden bei 37 °C für 15 min im Wasserbad aufgetaut, und es wurde die Protein Ca-Kofaktor-Aktivität von Protein S in Abwesenheit und in Anwesenheit von MAK49 gemessen (wie in Beispiel 2 beschrieben). Außerdem wurde in jeder Probe mit einem Immunoassay die Menge an freiem Protein S-Antigen bestimmt. Die Ergebnisse sind in Figur 3 vergleichend dargestellt.

Figur 3 zeigt für jede Probe die Protein Ca-Kofaktor-Aktivität von Protein S in Abwesenheit des Inhibitors MAK49 (gepunkteter Balken), die Menge an freiem Protein S-Antigen (längsgestreifte Balken) und den Quotienten aus der Protein Ca-Kofaktor-Aktivität von Protein S (Gerinnungszeit) gemessen in Abwesenheit des MAK49 und der Protein Ca-Kofaktor-Aktivität von Protein S (Gerinnungszeit) gemessen in Anwesenheit des MAK49 (quergestreifte Balken).

In Proben von Patienten mit einer Faktor V Leiden-Mutation kann Protein S seine Protein Ca-verstärkende Wirkung nicht entfalten, die Gerinnungsreaktion schreitet ungehindert voran, so dass eine zu kurze Gerinnungszeit und somit eine falsch zu niedrige Protein S-Aktivität bestimmt wird. Dies wird insbesondere durch den Vergleich mit der gemessenen Protein S-Antigen-Konzentration deutlich. Die Quotientenbildung gemäß dem erfindungsgemäßen Verfahren zeigt hingegen eine bessere Vergleichbarkeit mit den Ergebnissen der Protein S-Antigen-Bestimmung. Einzige Ausnahme ist eine Probe, die von einem homozygoten Faktor V Leiden-Patienten stammt, der zusätzlich mit einem oralen Antikoagulanz behandelt wird (Probe Nr. 4). Hier ist die Protein S-Aktivität sowohl im Protein Ca-Kofaktor-Aktivitäts-Test als auch im erfindungsgemäßen Verfahren mit Quotientenbildung gleichermaßen vermindert.

## Patentansprüche

1. Verfahren zur Bestimmung der Protein Ca-Kofaktor-Aktivität von Protein S in einer humanen oder tierischen Körperflüssigkeitsprobe, wobei
a. die Probe in einem ersten Reaktionsansatz vermischt wird mit
i. aktiviertem Protein C oder einer Substanz zur Aktivierung von Protein C und
ii. Faktor Va oder einer Substanz zur direkten oder indirekten Aktivierung von Faktor V
b. und wobei die Gerinnungsreaktion im Reaktionsansatz gemessen wird,
**dadurch gekennzeichnet, dass** die Probe in einem zweiten Reaktionsansatz zusätzlich zu denselben Komponenten, die zur Bereitsstellung des ersten Reaktionsansatzes verwendet werden, mit einem Inhibitor, der die Protein Ca-Kofaktor-Aktivität von Protein S inhibiert, vermischt wird und die Gerinnungsreaktion im Reaktionsansatz gemessen wird und dann die Protein Ca-Kofaktor-Aktivität von Protein S bestimmt wird, indem der Quotient aus der Gerinnungsreaktion, die im ersten Reaktionsansatz gemessen wurde, und der Gerinnungsreaktion, die im zweiten Reaktionsansatz gemessen wurde, gebildet wird.

2. Verfahren nach Anspruch 1, wobei die Probe im ersten und zweiten Reaktionsansatz zusätzlich mit Protein S Mangelplasma vermischt wird.

3. Verfahren nach einem der vorherigen Ansprüche, wobei das aktivierte Protein C, das mit der Probe vermischt wird, gereinigtes humanes Protein Ca ist.

4. Verfahren nach einem der Ansprüche 1-2, wobei die Substanz zur Aktivierung von Protein C Thrombin oder ein Protein C-Aktivator aus Schlangengift, bevorzugt aus Schlangengift der Gattung Agkistrodon contortrix, ist.

5. Verfahren nach einem der vorherigen Ansprüche, wobei die Substanz zur direkten Aktivierung von Faktor V Thrombin oder ein Faktor V-Aktivator aus Schlangengift, bevorzugt der Faktor V-Aktivator aus dem Schlangengift der Gattung Vipera russelli, ist.

6. Verfahren nach einem der Ansprüche 1-4, wobei die Substanz zur indirekten Aktivierung von Faktor V ein Kontaktphaseaktivator ist, bevorzugt ein Kontaktphaseaktivator aus der Gruppe Kaolin, Silica, Glas und Ellagsäure.

7. Verfahren nach einem der Ansprüche 1-4, wobei die Substanz zur indirekten Aktivierung von Faktor V Thromboplastin ist.

8. Verfahren nach einem der Ansprüche 1-4, wobei die Substanz zur indirekten Aktivierung von Faktor V ein Prothrombin-Aktivator ist, bevorzugterweise der Prothrombin-Aktivator aus dem Schlangengift der Gattung Echis carinatus.

9. Verfahren nach einem der Ansprüche 1-4, wobei die Substanz zur indirekten Aktivierung von Faktor V ein Faktor X-Aktivator ist, bevorzugterweise der Faktor X-Aktivator aus dem Schlangengift der Gattung Vipera russelli.

10. Verfahren nach einem der Ansprüche 1-4, wobei der Faktor Va, der mit der Probe vermischt wird, gereinigter humaner oder boviner Faktor Va oder gereinigter Faktor Va aus Kaninchen ist.

11. Verfahren nach einem der vorherigen Ansprüche, wobei der Inhibitor, der die Protein Ca-Kofaktor-Aktivität von Protein S spezifisch inhibiert, ein Protein S-bindender Antikörper oder ein Protein S-bindendes Antikörperfragment oder C4b-binding-Protein ist.

12. Verfahren nach Anspruch 11, wobei der Inhibitor, der die Protein Ca-Kofaktor-Aktivität von Protein S spezifisch inhibiert, ein Protein S-bindender Antikörper ist, der von der Hybridomazelllinie DSM ACC3188 produziert wird, oder ein Protein S-bindendes Fragment davon.

13. Verfahren nach einem der vorherigen Ansprüche, wobei die Gerinnungsreaktion im ersten und im zweiten Reaktionsansatz durch Bestimmung des Gerinnungszeitpunkts gemessen wird.

## Claims

1. Method for determining the protein Ca-cofactor activity of protein S in a human or animal body fluid sample, wherein
a. the sample is mixed, in a first reaction volume, with
i. activated protein C or a substance for activating protein C and
ii. factor Va or a substance for directly or indirectly activating factor V
b. and wherein the coagulation reaction is measured in the reaction volume,
**characterized in that** the sample is mixed, in a second reaction volume, with an inhibitor which inhibits the protein Ca-cofactor activity of protein S, in addition to the same components which are used for providing the first reaction volume, and the coagulation reaction is measured in the reaction volume, and then the protein Ca-cofactor activity of protein S is determined by calculating the quotient of the coagulation reaction measured in the first reaction volume and the coagulation reaction measured in the second reaction volume.

2. Method according to Claim 1, wherein the sample in the first and the second reaction volume is additionally mixed with protein S-deficient plasma.

3. Method according to any of the preceding claims, wherein the activated protein C mixed with the sample is purified human protein Ca.

4. Method according to either of Claims 1 and 2, wherein the substance for activating protein C is thrombin or a protein C activator from snake venom, preferably from snake venom from the species *Agkistrodon contortrix.*

5. Method according to any of the preceding claims, wherein the substance for directly activating factor V is thrombin or a factor V activator from snake venom, preferably the factor V activator from snake venom from the species *Vipera russelli .*

6. Method according to any of Claims 1-4, wherein the substance for indirectly activating factor V is a contact phase activator, preferably a contact phase activator from the group consisting of kaolin, silica, glass and ellagic acid.

7. Method according to any of Claims 1-4, wherein the substance for indirectly activating factor V is thromboplastin.

8. Method according to any of Claims 1-4, wherein the substance for indirectly activating factor V is a prothrombin activator, preferably the prothrombin activator from the snake venom from the species *Echis carinatus.*

9. Method according to any of Claims 1-4, wherein the substance for indirectly activating factor V is a factor X activator, preferably the factor X activator from the snake venom from the species *Vipera russelli.*

10. Method according to any of Claims 1-4, wherein the factor Va mixed with the sample is purified human or bovine factor Va or purified factor Va from rabbits.

11. Method according to any of the preceding claims, wherein the inhibitor which specifically inhibits the protein Ca-cofactor activity of protein S is a protein S-binding antibody or a protein S-binding antibody fragment or C4b-binding protein.

12. Method according to Claim 11, wherein the inhibitor which specifically inhibits the protein Ca-cofactor activity of protein S is a protein S-binding antibody produced by the hybridoma cell line DSM ACC3188, or a protein S-binding fragment thereof.

13. Method according to any of the preceding claims, wherein the coagulation reaction is measured in the first and the second reaction volume by determining the coagulation time.

## Revendications

1. Procédé de détermination de l'activité de la protéine Ca-cofacteur de la protéine S dans un échantillon de liquide corporel humain ou animal, dans lequel
a. on mélange l'échantillon en une première masse réactionnelle à
i. de la protéine C activée ou une substance d'activation de la protéine C, et
ii. du facteur Va ou une substance d'activation directe ou indirecte du facteur V
b. et dans lequel on mesure la réaction de coagulation dans la masse réactionnelle,
**caractérisé en ce que** l'on mélange l'échantillon dans une deuxième masse réactionnelle supplémentairement aux mêmes constituants qui sont utilisés pour préparer la première masse réactionnelle à un inhibiteur qui inhibe l'activité protéine Ca-cofacteur de la protéine S et on mesure la réaction de coagulation dans la masse réactionnelle et on détermine, ensuite, l'activité protéine Ca-cofacteur de la protéine S en formant le quotient de la réaction de coagulation qui a été mesurée dans la première masse réactionnelle par la réaction de coagulation qui a été mesurée dans la deuxième masse réactionnelle.

2. Procédé suivant la revendication 1, dans lequel on mélange l'échantillon dans la première et la deuxième masse réactionnelle supplémentairement à du plasma ayant une carence en protéine S.

3. Procédé suivant l'une des revendications précédentes, dans lequel la protéine C activée, qui est mélangée à l'échantillon, est de la protéine Ca humaine purifiée.

4. Procédé suivant l'une des revendications 1 à 2, dans lequel la substance d'activation de la protéine C est de la thrombine de protéine C ou un activateur de protéine C d'un venin de serpent, de préférence d'un venin de serpent de l'espèce Agkistrodon contortrix.

5. Procédé suivant l'une des revendications précédentes, dans lequel la substance d'activation directe du facteur V est de la thrombine ou un activateur de facteur V provenant d'un venin de serpent, de préférence l'activateur du facteur V provenant du venin de serpent de l'espèce Vipera russelli.

6. Procédé suivant l'une des revendications 1 à 4, dans lequel la substance d'activation indirecte du facteur V est un activateur de phase de contact, de préférence un activateur de phase de contact du groupe du kaolin, de la silice, du verre et de l'acide ellagique.

7. Procédé suivant l'une des revendications 1 à 4, dans lequel la substance d'activation indirecte du facteur V est de la thromboplastine.

8. Procédé suivant l'une des revendications 1 à 4, dans lequel la substance d'activation indirecte du facteur V est un activateur de la prothrombine, de préférence activateur de la prothrombine provenant du venin de serpent de l'espèce Echis carinatus.

9. Procédé suivant l'une des revendications 1 à 4, dans lequel la substance d'activation indirecte du facteur V est un activateur du facteur X, de préférence un activateur du facteur X provenant du venin de serpent de l'espèce Vipera russelli.

10. Procédé suivant l'une des revendications 1 à 4, dans lequel le facteur Va, qui est mélangé à l'échantillon, est du facteur Va humain ou bovin purifié ou du facteur Va purifié de lapin.

11. Procédé suivant l'une des revendications précédentes, dans lequel l'inhibiteur qui inhibe spécifiquement l'activité de la protéine Ca-Cofacteur de la protéine S, est un anticorps se fixant à la protéine S ou un fragment d'anticorps se fixant à la protéine S, ou une protéine fixant C4b.

12. Procédé suivant la revendication 11, dans lequel l'inhibiteur qui inhibe spécifiquement l'activité protéine Ca-cofacteur de la protéine S, est un anticorps se fixant à la protéine S qui est produit par la lignée de cellules hybridomes DSM ACC3188 ou un fragment de celle-ci se fixant à la protéine S.

13. Procédé suivant l'une des revendications précédentes, dans lequel on mesure la réaction de coagulation dans la première et dans la deuxième masse réactionnelle en déterminant l'instant de coagulation.
